# EUROPÄISCHE PATENTSCHRIFT

(11) **EP 1 095 008 B1**
(45) Veröffentlichungstag und Bekanntmachung des Hinweises auf die Patenterteilung: **01.10.2003**
(21) Anmeldenummer: 99932786.9
(22) Anmeldetag: 02.07.1999
(51) Int. Cl.: C07C 209/84, C07C 209/48

(54) **VERFAHREN ZUR REINIGUNG VON HEXAMETHYLENDIAMIN AUS SEINEN MISCHUNGEN MIT EINEM UNGESÄTTIGTEN CYCLISCHEN IMIN**
METHOD FOR PURIFYING HEXAMETHYLENEDIAMINE IN MIXTURES OF HEXAMETHYLENEDIAMINE AND AN UNSATURATED CYCLIC IMINE
PROCEDE DE PURIFICATION D'HEXAMETHYLENEDIAMINE DANS UN MELANGE HEXAMETHYLENEDIAMINE-IMINE CYCLIQUE INSATUREE

(30) Priorität: 09.07.1998 DE 19830598
(43) Veröffentlichungstag der Anmeldung: 02.05.2001
(73) Patentinhaber: BASF AKTIENGESELLSCHAFT, 67056 Ludwigshafen (DE)
(72) Erfinder: MERK, Claudia, D-67117 Limburgerhof (DE); BASSLER, Peter, D-68519 Viernheim (DE); FISCHER, Rolf, D-69121 Heidelberg (DE); VOIT, Guido, D-67251 Freinsheim (DE); LUYKEN, Hermann, D-67069 Ludwigshafen (DE)
(86) Internationale Anmeldenummer: EP9904584
(87) Internationale Veröffentlichungsnummer: WO00002842

(56) Entgegenhaltungen:
- EP-A- 0 497 333
- WO-A-93/02230
- CH-A- 393 353

## Beschreibung

Die vorliegende Erfindung betrifft ein Verfahren zur Reinigung von Hexamethylendiamin in einer Mischung enthaltend Hexamethylendiamin und Tetrahydroazepin durch elektrochemische Umwandlung von Tetrahydroazepin in Gegenwart von solvatisierten Protonen in Azepan bei einer Stromdichte im Bereich von 1 to 30 mA/cm² and einer Spannung im Bereich von 14,4 bis 58 V.

Es ist allgemein bekannt, beispielsweise aus Weissermel/Arpe, Industrielle Organische Chemie, Verlag Chemie, dritte Auflage, 1988, Seite 266, und WO-A-96/20166 Adipodinitril im Gegenwart von Ammoniak unter Hochdruckbedingungen an heterogenen Katalysatoren zu 6-Aminocapronitril (ACN) und/oder Hexamethylendiamin (HMD) , die beide wichtige Vorprodukte für die Herstellung von Polyamiden wie Nylon 6 und Nylon 6.6 darstellen, zu hydrieren.

Je nach Katalysator entstehen dabei in unterschiedlichen Mengen unerwünschte Nebenprodukte, wie Tetrahydroazepin (THA) , 1-Amino-2-cyanocyclopenten (ICCP), 2-Aminomethylcyclopentylamin <AMCPA), 1,2-Diaminocyclohexan (DCH) und Bishexamethylentriamin (BHMTA), die sich - im Gegensatz zu dem Nebenprodukt Azepan (Hexamethylenimin; HMI) - nur mit hohem Aufwand von dem als Wertprodukt entstehenden ACN und/oder HMD abtrennen lassen.

So ist aus US-A-4.282.381, Spalte 2, Tabelle 1, bekannt, daß bei Hydrierung von Adipodinitril zu HMD in Gegenwart von Eisenkatalysatoren als Nebenprodukt unter anderem durchschnittlich 200 bis 900 ppm Tetrahydroazepin entstehen.

EP-A-497 333 offenbart die Reinigung eines aliphatischen Amins durch die Entfernung eines cyclischen, aliphatischen, ungesättigten Amins mittels einer Lauge und nachfolgender Destillation.

Aus CH-393 353 ist bekannt, daß solche Verunreinigungen elektrochemisch bei Spannungen im Bereich zwischen 1,3 und 1,5 V reduziert werden können; dieses Verfahren sei jedoch nicht industriell anwendbar.

In WO-A-93/02230 wird die elektrochemische Reduzierung von Nitrilen, wie Adipodinitril, zu Aminen, wie Hexamethylendiamin oder Aminocapronitril, beschrieben. Die Spannung beträgt gemäß den Beispielen 1,5 V bzw. 12,3 V. Die Stromdichte beträgt 75 A/cm². Ein Hinweis auf die Reinigung von HMD durch Entfernung von THA ist WO-A-93/02230 nicht zu entnehmen.

Große Mengen an THA führen zu einem hohen Reinigungsaufwand, z.B. als Destillationsaufwand, der sich in beträchtlichen Investitions- und Energiekosten oder hohem Chemikalienverbrauch insbesondere durch komplexe Hydride wie NaBH₄ oder LiAlH₄ niederschlägt.

Aufgabe der vorliegenden Erfindung war daher, ein Verfahren zur Verfügung zu stellen, das die Reinigung von Hexamethylendiamin in Mischungen enthaltend HMD und THA auf technisch einfache und wirtschaftliche Weise ermöglicht.

Demgemäß wurde das eingangs definierte Verfahren gefunden.

Dabei wird THA erfindungsgemäß in Mischungen enthaltend HMD und THA eingesetzt. Solche Mischungen können beispielsweise bei der bereits genannten Hydrierung von Adipodinitril erhalten werden.

Die Gehalte an THA bezogen auf HMD betragen dabei üblicherweise 100 bis 2500, insbesondere 200 bis 900 ppm.

Eine Mischung enthaltend HMD und THA kann zu der Umsetzung in reiner Form oder vorzugsweise in Mischungen mit flüssigen Verdünnungsmitteln eingesetzt werden.

Als flüssige Verdünnungsmittel kommen vorteilhaft Hydroxyl-Gruppen enthaltende Verbindungen, vorzugsweise Wasser oder Alkohole mit 1 bis 4 Kohlenstoffatomen, wie Methanol, Ethanol, Propan-1-ol, Propan-2-o1, Butan-1-ol, Butan-2-ol, Methylpropan-2-ol, Methylpropan-1-ol, vorzugsweise Wasser oder Methanol, insbesondere Wasser, oder deren Gemische in Betracht, sowie Gemische solcher Hydroxyl-Gruppen enthaltenden Verbindungen mit Nicht-Hydroxyl-Gruppen enthaltenden Verbindungen, vorzugsweise Etherh, wie Dimethoxyethan oder Tetrahydrofuran oder deren Gemische.

Die Menge an flüssigem Verdünnungsmittel kann durch einige einfache Vorversuche leicht ermittelt werden.

Bei der Verwendung einer Mischung aus HMD und THA mit einem THA-Gehalt von zwischen 100 und 2500, insbesondere 200 und 900 ppm, hat sich ein Gehalt an Wasser und/oder Methanol, insbesondere Wasser, von 0.01 bis 20 Gew.-% bezogen auf die gesamte Mischung als besonders vorteilhaft erwiesen.

Für die elektrochemische Umsetzung eignen sich Elektrolysezellen mit einer, vorzugsweise mehreren, wie 2, 3 oder 4, insbesondere 2 Zellkammern. Werden mehrere Zellkammem eingesetzt, so trennt man zweckmäßigerweise die Kammern gegeneinander durch Ionen-durchlässige Membranen.

Im Falle von 2 Kammern kommen hierzu insbesondere Kationen-durchlässige, insbesondere Protonen-durchlässige Membranen in Betracht.

Solche Membranen sind allgemein bekannt und beispielsweise unter dem Handelsnamen Nafion, beispielsweise Nafion 324 (Firma DuPont) kommerziell verfügbar.

In den Anodenraum gibt man vorteilhaft eine Flüssigkeit, die bei. Anlegen einer Spannung an die Elektrolysezelle anodenseitig solvatisierte Protonen bilden kann. Hierzu kommen Hydroxyl-Gruppen enthaltende Verbindungen, wie Wasser oder Säuren, vorzugsweise organische Säuren, wie Mono- oder Dicarbonsäuren, oder deren Salze, sowie deren Gemische in Betracht. Zur Erhöhung der elektrischen Leitfähigkeit kann man diesen Flüssigkeiten organische oder anorganische Basen, wie HMD vorzugsweise anorganische Säuren, wie Schwefelsäure, insbesondere organische Säuren, wie Adipinsäure, in Mengen von 0,1 bis 2 Gew.-% bezogen auf die gesamte Flüssigkeit beimischen.

Derartige Flüssigkeiten bilden üblicherweise bei Anlegen einer Spannung an die Elektrolysezelle solvatisierte Protonen unter gleichzeitiger Entwicklung von Sauerstoff.

Anodenmaterialien für diese Teitreaktion der Elektrolysezelle sind an sich bekannt und kommerziell verfügbar. Vorteilhaft eingesetzt werden können beispielsweise eine Ru-Ta-Ti-Mischoxid oder eine Ir-Ti-Mischoxid, wie sie beispielsweise als Dendra DSA Elektrode oder Heraeus Pita 64 kommerziell verfügbar sind.

In den Kathodenraum der Elektolysezelle bringt man eine Mischung enthaltend HMD und THA.

Zur Erhöhung der elektrischen Leitfähigkeit können in den Kathodenraum organische, vorzugsweise anorganische Salze, beispielsweise Alkalihalogenide wie Lithiumchlorid, anorganische oder organische Basen oder anorganische oder organische Säuren, beispielsweise Monocarbonsäure, vorzugsweise Dicarbonsäuren, insbesondere Adipinsäure, in Mengen von 0,1 bis 2 Gew.-% bezogen auf die gesamte Menge an Katholyt gegeben werden.

Vorteilhaft kann in den Katholyten ein für die Reaktion katalytisch aktives, vorzugsweise in Bezug auf den Katholyten heterogenes Material, wie feinverteiltes Metall, vorzugsweise Eisen, Raney-Cobalt oder Raney-Nickel, insbesondere Raney-Mickel, in Mengen von 1 bis 20 Gew.-% bezogen auf den Katholyten gegeben werden. Solche katalytische aktiven Materialien können nach der Reaktion in an sich bekannter und einfacher Weise, wie durch Filtration, von der Mischung abgetrennt werden.

Filtermaterial der Kathode für diese Teilreaktion der Elektrolysezelle sind an sich bekannt und kommerziell verfügbar. Vorteilhaft eingesetzt werden können Metallfilter wie Edelstahl-Filter oder Filter aus platiniertem Titan.

Die Reaktion führt man üblicherweise bei einer Temperatur durch, bei der Anolyt und Katholyt in flüssiger Form vorliegen, vorzugsweise bei 10 bis 60, insbesondere 36 bis 40°C durch.

Die Stromdichte beträgt erfindungsgemäß 1 bis 30 mA/cm², vorzugsweise 15 bis 30 mA/cm², wodurch sich Spannungen von 14,4 bis 58 V, vorzugsweise 14,4 bis 36 V ergeben.

Aus der erfindungsgensäß eingesetzten Mischung enthaltend HMD und THA kann das entstehende Azepan von der Produktmischung in an sich bekannter Weise, wie durch Destillation, Kristallisation oder Extraktion, abgetrennt werden.

### Beispiel

Die Beispiele wurden unter folgenden Bedingungen durchgeführt:

**Tabelle 1**

| | |
|---|---|
| Zelltyp | Geteilte Zelle, 2 Kammern |
| Anode | Heraeus Pita 64, sauerstoffentwickelnd |
| Kathode | Kantenspaltelement aüs platiniertem Titan, Spaltbreite: 10.0 µm |
| Elektrodenfläche | 100 cm² |
| Membran | Nafion 324. |
| Elektrolysetemperatur | 38°C |
| Anolyt | 1200 g wäßrige 1 %-ige Schwefelsäure |
| Katholyt | Katalysator: 50 g Raney-Nickel Rest: s. Tabelle 2 |

**Tabelle 2**

| | | | | | |
|---|---|---|---|---|---|
| HMD [g] | 1120 | 840 | 1120 | 1120 | 1120 |
| Methanol [g] | - | 560 | - | - | - |
| H₂O [g] | 280 | - | 280 | 280 | 180 |
| LiCl [g] | - | 28 | - | - | - |
| Adipinsäure [g] | - | - | - | 21 | 21 |
| Stromdichte [mA/cm²] | 1,6-2, 9 | 30 | 0,5-0,9 | 15 | 15 |
| U (Anfang) [V] | 58,0 | 14,4 4 | 57,9 9 | 36,8 8 | 47,6 |
| U (Ende) [V] | 58,1 | 25,7 | 56,3 | 22,9 | 20,3 |
| THA Anfang [ppm] | 1399 | 753 | 1530 | 775 | 829 |
| THA Ende [ppm] | 91/30 | 37 | 81/41 | 185/62/46 | 196/55/15 |
| Zeit [h] | 10/50 | 4 | 10/43 | 5/10/20 | 5/10/15 |
| Reduzierung THA [%] | 93/98 | 95 | 95/97 | 76/92/94 | 76/93/98 |

## Patentansprüche

1. Verfahren zur Reinigung von Hexamethylendiamin in einer Mischung enthaltend Hexamethylendiamin und Tetrahydroazepin durch elektrochemische Umwandlung von Tetrahydroazepin in Gegenwart von solvatisierten Protonen in Azepan bei einer Stromdichte im Bereich von 1 to 30 mA/cm³ und einer Spannung im Bereich von 14.4 bis 58 V.

2. Verfahren nach Anspruch 1, wobei man die Umsetzung in Gegenwart von Wasser oder einem Alkohol mit 1 bis 4 Kohlenstoffatomen durchführt.

3. Verfahren nach Anspruch 1 oder 2, wobei man die Umsetzung in Gegenwart eines Katalysators durchführt.

4. Verfahren nach Anspruch 3, wobei man als Katalysator Raney-Nickel einsetzt.

5. Verfahren nach den Ansprüchen 1 bis 4, wobei man die Umsetzung in Gegenwart einer organischen Säure durchführt.

6. Verfahren nach Anspruch 5, wobei man als organische Säure eine Mono- oder Dicarbonsäure einsetzt.

7. Verfahren nach Anspruch 6, wobei man als Dicarbonsäure Adipinsäure einsetzt.

## Claims

1. The process for purifying hexamethylenediamine in a mixture comprising hexamethylenediamine and tetrahydroazepine by electrochemical conversion of tetrahydroazepine in the presence of solvated protons into perhydroazepine at a current density from 1 to 30 mA/cm³ and a voltage from 14.4 to 58 V.

2. The process of claim 1, wherein the conversion is carried out in the presence of water or of an alcohol having from 1 to 4 carbon atoms.

3. The process of claim 1 or 2, wherein the conversion is carried out in the presence of a catalyst.

4. The process of claim 3, wherein the catalyst used is Raney nickel.

5. The process of any of claims 1 to 4, wherein the conversion is carried out in the presence of an organic acid.

6. The process of claim 5, wherein the organic acid used is a mono- or dicarboxylic acid.

7. The process of claim 6, wherein the dicarboxylic acid used is adipic acid.

## Revendications

1. Procédé de purification d'hexaméthylènediamine dans un mélange contenant de l'hexaméthylènediamine et de la tétrahydroazépine par conversion électrochimique de la tétrahydroazépine en azépanne en présence de protons solvatés, avec une densité de courant de l'ordre de 1 à 30 mA.cm³ et une tension de l'ordre de 14,4 à 58 V.

2. Procédé selon la revendication 1, dans lequel on entreprend la réaction en présence d'eau ou d'un alcool comportant de 1 à 4 atomes de carbone.

3. Procédé selon la revendication 1 ou 2, dans lequel on entreprend la réaction en présence d'un catalyseur.

4. Procédé selon la revendication 3, dans lequel on met en oeuvre comme catalyseur du nickel de Raney.

5. Procédé selon les revendications 1 à 4, dans lequel on entreprend la réaction en présence d'un acide organique.

6. Procédé selon la revendication 5, dans lequel on met en oeuvre comme acide organique un acide mono- ou dicarboxylique.

7. Procédé selon la revendication 6, dans lequel on met en oeuvre comme acide dicarboxylique de l'acide adipique.
